**Europäisches Patentamt**

**European Patent Office**

**Office européen des brevets**

(11) Publication number: **0 111 388**

**B1**

# EUROPEAN PATENT SPECIFICATION

(45) Date of publication of patent specification: **25.03.87**

(21) Application number: **83306725.9**

(22) Date of filing: **04.11.83**

(51) Int. Cl.⁴: **C 07 F 17/02, A 61 K 31/80, A 61 K 31/295**

(54) **Therapeutic compounds of platinum and palladium and their preparation.**

(30) Priority: **10.11.82 US 440449**
**14.10.83 US 540871**

(43) Date of publication of application:
**20.06.84 Bulletin 84/25**

(45) Publication of the grant of the patent:
**25.03.87 Bulletin 87/13**

(84) Designated Contracting States:
**AT BE CH DE FR GB IT LI LU NL SE**

(56) References cited:
**GB-A-1 531 211**
**GB-A-1 541 436**
**US-A-4 356 166**

**CHEMICAL ABSTRACTS, vol. 98, no. 14, 4th April 1983, page 377, no. 113588q, Columbus, Ohio, US E. HURWITZ: "Attempts at site-directed experimental chemotherapy with antibody drug-conjugates"**

**INORGANIC CHEMISTRY, vol. 16, no. 6, 1977, pages 1525-1529. B. LIPPERT et al.: "CIS-dinitratodiammineplatimum(II), CIS-Pt(NH3)2(NO3)2. Crystalline structure and vibrational spectra"**

(73) Proprietor: **Inco Alloys International, Inc.**
**Huntington West Virginia 25720 (US)**

(72) Inventor: **Smith, Gaylord Darrell**
**216 Summit Avenue**
**Ramsey, NJ 07446 (US)**
Inventor: **Brown, Douglas Scott**
**Maplebrook Road**
**Tuxedo, NY 10987 (US)**
Inventor: **Bernstein, Philip**
**16 Stonehouse Road**
**Glen Ridge, NY 07028 (US)**
Inventor: **Weller, John Edward**
**45 Riverview Street**
**Walden, NY 12586 (US)**

(74) Representative: **Greenstreet, Cyril Henry et al**
**Haseltine Lake Partners Motorama Haus 502**
**Rosenheimer Strasse 30**
**D-8000 München 80 (DE)**

(56) References cited:
**NATO ADVANCES STUDY INST. SER., ser. A, vol. 47, 1982, pages 31-54, Targeting drugs. R. ARNON: "Antibodies and dextran as anti-tumor drug carriers"**

**Chemical - Biological Interations, volume 7, pages 305 - 315, 1973**

## Description

This invention relates to new compositions of matter containing platinum or palladium that are useful for combatting cancer cell and trypanosomal infection in mammals.

The compound *cis*-dichlorodiamine platinum (II) (cisplatin) has become well known in the treatment of various cancers in mammals, but it is toxic and hence can be used only in low dosage. It would be desirable to provide an effective product which could be tolerated in a mammalian body in larger doses and would exhibit a longer half-life. It would also be desirable to provide an effective composition which possessed enhanced solubility in water and serum in comparison to cisplatin.

It has been proposed in GB—A 1,541,436 to link certain specified organic cytotoxic drugs having free amino or carboxyl groups by a peptide or other covalent link to polymer carriers having a molecular weight from 5,000 to 500,000 and suitable for binding to immuno-globulin, so that the cytotoxic drug is cleaved enzymatically on the target cell surface.

It has also been suggested by C. R. Morris and G. R. Gale in "Chem. Biol. Interactions", 7 (1973) 305—315, that the compounds *cis*-dichloro(dipyridine) platinum (II) and *cis*-Pt (II) may associate with electron-rich areas of nucleic acids and proteins, and also with body pools of free nucleotides and amino acids, but no such compositions were isolated or specifically identified.

The present invention is based on the discovery that members of a novel group of platinum and palladium compositions exhibit desirable advantages set out above and/or are effective against trypanosomal infections.

The novel compositions according to the invention comprise water-soluble anionic organic macromolecules, namely anionic polysaccharides, anionic polyamino acids or enzymes having anionic functionality (e.g. enzymes having anionic side-chains), having a molecular weight from 5000 to 60,000, combined with a moiety of square planar coordination about the metal atom M, having the formula [*cis* MLL']$^{++}$, wherein M is divalent platinum or divalent palladium and L and L' are ammonia or mono- or difunctional primary amines, so as to satisfy at least part of the valence requirements of the anionic functional groups of the anionic macromolecules. The invention includes pharmaceutical compositions comprising such compositions.

The compositions are not specific compounds having readily determinable structures, but rather mixtures of related polymeric materials in which a plurality of the square planar platinum or palladium coordination moieties are either carried on one anionic macromolecule or serve as a bridge or bridges between two anionic macromolecules. Where multiple bridging occurs, more than two macromolecules can be linked together. In the ordinary case, the compositions will contain at least 2% by weight of platinum or palladium bonded onto the macromolecule (based on the weight of metal plus macromolecule).

By a "water-soluble organic macromolecule" is meant one which exhibits solubility in water when combined with cations such as $H^+$, $NH_4^+$, alkali metals, calcium and magnesium.

Examples of anionic macromolecular materials which are of suitably low toxicity and can be employed as the bases for the compositions of the present invention include (but are not limited to) poly-L-glutamic acid (PGA), carboxymethyl cellulose, ferritin, chondroitin sulphate, poly-L-aspartic acid, dextran sulphate, poly[divinyl ether-co-maleic anhydride], poly[acrylic acid co-maleic anhydride], polyacrylic acid, serum albumin and desulphated heparin.

The ammonia or substituted ammonia groups L and L' in the coordinated platinum or palladium moiety may in particular be identical or be two primary amine functional groups present on a single molecule. Representative primary amines which can be used in compositions of the invention include isoamylamine, pentylamine, cyclohexyl amine, 1,2-diaminocyclohexane, and ethylenediamine.

The novel compositions may be prepared by reacting an aqueous solution of the anionic macromolecule, which may be in the salt form, e.g. the sodium salt form, with an aqueous solution of an aminated *cis*-platinum or *cis*-palladium compound in aquated nitrate form, $MLL'(H_2O)_2(NO_3)_2$ i.e. in aquated salt form. The platinum or palladium compound may be prepared by the method of Lippert, Lock, Rosenberg and Zvagulis, *Inorganic Chemistry*, Vol. 16, No. 6, 1977, pp. 1525—1529. The reaction mixture is stirred for a considerable time period, e.g. 16 hours or more, and the product is purified by dialysis or filtration, depending upon whether or not the product is soluble in the reaction medium.

Using as a basis a macromolecular entity that is water soluble, it is possible to produce both water-soluble and water-insoluble platinum- or palladium-containing compositions. As a general rule, if more than about 80% of the free anionic functional groups (e.g. carboxylic acid groups) on a macromolecule are complexed by the [*cis* MLL']$^{++}$ moiety, the resulting composition of the present invention will be insoluble in water or sera owing to both the low solubilising characteristic of the [*cis* MLL']$^{++}$ moiety compared to say, the sodium ion, and also to the fact that at this high level of precious metal content, the incidence of crosslinking of macromolecules to form even larger macromolecules is inherently high. There are however a number of exceptions to this rule of an upper limit of 80% complexing for water- or sera-solubility. First, a macromolecule of very high molecular weight might only tolerate a maximum of say 50% or 60% of its anionic functional units being complexed to provide an end product which is water- or plasma-soluble. Second, if the primary amines $RNH_2$ or $NH_2R'NH_2$ in the [*cis* MLL']$^{++}$ moiety are such that the R or the R' group is significantly hydrophobic the macromolecule having anionic functionality will tolerate less complexing by such a $[cis\ MLL']^{++}$ moiety with the final product retaining water-solubility, than it would tolerate where L and L' are $NH_3$ or are the groups of $NH_2CH_2CH_2NH_2$.

The compositions of the present invention are thus divided by character into water-soluble and water-insoluble species. The functions of these species differ significantly when the compositions are employed as drugs for treating cancers and tumours. The water-soluble species are adapted to be injected into mammalian body fluids, e.g. blood, and, being soluble in the plasma thereof, are adapted to be carried throughout the mammalian body where that fluid flows, absent a biofunctional barrier to the composition. Water-solubility is also advantageous for the treatment of trypanosomal infections. Water-insoluble compositions of the invention on the other hand are readily adaptable to be implanted in tumourous tissue. Such implanted drug compositions can be expected to release relatively high concentrations of therapeutically effective species by hydrolysis, by minimal solubility, by enzymatic attack, or by a combination of all three means in a very local volume thereby directly attacking the cancerous mass.

Some examples will now be given, in which the abbreviations have the following meanings:

DACH = "1,2-diaminocyclohexane"
PGA = "polyglutamic acid"
en = "ethylenediamine".

The 1,2-diaminocyclohexane employed in the preparations was obtained from the Aldrich Chemical Co., as an 85% technical grade and comprised a mixture of cis and trans isomers with the trans isomer containing both plus and minus optical enantiomers.

Example I
A. Preparation of cis-Pt[(DACH)($H_2O$)$_2$]($NO_3$)$_2$

Into a 250 ml round bottom flask 16.4 g (0.0431 moles) of pure $cis$-Pt(DACH)(Cl)$_2$ was charged.

A silver nitrate solution was prepared by dissolving 14.3 g (0.0844 moles) of AgNO$_3$ in 100 ml of distilled water. The solution was then charged into the flask and stirred in the dark at 60°C for thirty minutes. The suspension was then cooled to room temperature while stirring an additional sixteen hours. Next, the reaction was filtered, resulting in a clear pale yellow aqueous solution containing Pt[(DACH)($H_2O$)$_2$]($NO_3$)$_2$ solution. Other nitrato platinum (II) and palladium (II) solutions described hereinafter were prepared in a similar manner.

B. Preparation of cis-Pt(DACH) complexed with dextran sulphate

In a 50 ml round bottom flask 0.5 g of dextran sulphate-sodium salt (average molecular weight 5,000) was dissolved in 20 ml of distilled water. Next, 2.0 ml of a 0.2615 M Pt[(DACH)($H_2O$)$_2$]($NO_3$)$_2$ aqueous solution prepared as described in I(A) was slowly added with a syringe to the polymer solution. The reaction was stirred for 16 hours.

The sample was then purified by dialysis over a six hour period (dialysis tubing possessed a molecular weight cut-off of 5,000—6,000). Analysis of the purified sample revealed a 0.19% weight content of platinum in solution. The dialysis product was used in the testing described in Table I.

A portion of the dialysis product was lyophilized to produce a fluffy, crystalline-appearing, golden solid. Lyophilization was accomplished over one week at −60°C, with 10 microns vacuum. The solid product was reconstitutable in distilled water.

Example II
Preparation of cis-Pd(DACH) complexed with PGA

In a 50 ml round bottom flask 1.0 g (0.0066 moles in terms of polymer units) of poly-L-glutamic acid-sodium salt was dissolved in 20 ml of distilled water. With stirring 14.0 ml of a 0.1447 M $cis$-Pd[(DACH)($H_2O$)$_2$]($NO_3$)$_2$ aqueous solution was slowly added with an addition funnel to the polymer solution. The reaction was stirred for 16 hours.

The sample was then purified by dialysis over a sixteen hour period (dialysis tubing possessed a molecular weight cut-off of 6,000). Analysis of the purified sample revealed a 0.22% weight content of palladium in solution.

Example III
Preparation of cis-Pt(DACH) complexed with PGA

In a 50 ml round bottom flask 0.5 g (0.0033 moles in terms of polymer units) of poly-L-glutamic acid-sodium salt (average molecular weight 60,000) was dissolved in 20 ml of distilled water. With stirring 2.0 ml of a 0.2516 M $cis$-Pt[(DACH)($H_2O$)$_2$]($NO_3$)$_2$ aqueous solution was slowly added with a syringe to the polymer solution. The reaction was stirred for 16 hours.

The sample was then purified by dialysis over a six hour period (dialysis tubing possessed a molecular weight cut-off of 6,000). Analysis of the purified sample revealed a 0.24% weight content of platinum in solution. A portion of the dialysis product was lyophilized as in Example I(B) to produce a fluffy, crystalline-appearing, light-golden solid. Redissolution of the solid in distilled water was accomplished.

Example IV
Preparation of cis-Pt($NH_3$)$_2$ complexed with PGA

In a 50 ml round bottom flask 1.0 g (0.0066 moles in terms of polymer units) of poly-L-glutamic acid-solution salt (average molecular weight 60,000) was dissolved in a 30 mls of distilled water. Next, 7.8 ml of a 0.2537 M $cis$-Pt[(NH$_3$)$_2$(H$_2$O)$_2$](NO$_3$)$_2$ aqueous solution was slowly added with a syringe to the polymer solution. The reaction was stirred for 16 hours.

The sample was then purified by dialysis over a 60 hour period (dialysis tubing possessed a molecular weight cut-off of 5—6,000). Analysis of the purified sample revealed a 0.61% weight content of platinum in solution (24.2% of the free carboxyl side chains on PGA being complexed to platinum).

Example V
Cis-Pt(DACH) complexed with chondroitin sulphate

In a 250 ml round bottom flask 2.0 g of chondroitin sulphate-sodium salt was dissolved in 80 ml of distilled water. With stirring 8.0 ml of 0.3405 M $cis$-Pt[(DACH)(H$_2$O)$_2$](NO$_3$)$_2$ aqueous solution was slowly added to the polymer solution. The reaction was stirred for 16 hours. Overnight the reaction product had partially turned to gel.

The sample was then purified by dialysis over a six hour period (dialysis tubing possessed a molecular weight cut-off of 6,000—8,000). Analysis of the purified sample revealed a 0.455% weight content of platinum in solution.

Example VI
Preparation of cis-Pt(NH$_3$)$_2$ complexed with bovine serum albumin

In a 50 ml round bottom flask 1.0 g of bovine serum albumin (prepared using method IV of Cohn, E. G. et al., *J. Am. Chem. Soc.,* 1947 *69* (1953)) was dissolved in 25 ml of distilled water. Next, 7.8 ml of 0.2537 M $cis$-Pt[(NH$_3$)$_2$(H$_2$O)$_2$](NO$_3$)$_2$ aqueous solution was slowly added with a syringe to the solution of serum albumin. The reaction was stirred for 16 hours.

The sample was then purified by dialysis over a six hour period (dialysis tubing possessed a molecular weight cut-off of 6,000). Analysis of the purified sample revealed a 0.51% weight content of platinum in solution.

Example VII
In vivo tests against trypanosomes

*In vitro* tests against *T. Brucei* indicated that the compositions $cis$-Pt(DACH) complexed with (PGA) and $cis$-Pt(NH$_3$)$_2$ complexed with (PGA) were immediately effective at low dosage levels. Accordingly, *in vivo* testing was conducted in mice.

The mice selected were NCS male mice weighing 20 to 25 g. Five animals were used for each dosage level. A count of 50,000 *T. Congolense* was administered intraperitoneally to each animal. At this dosage, control animals given no anti-trypanosomal treatment died in 5 to 8 days. One day after the injection of trypanosomes, the platinum drug of the invention was administered. In one test at 15 mg Pt/kg, $cis$-Pt(DACH) complexed with (PGA) gave a 20% cure rate, and in another test at 6.25 mg Pt/kg, the same compound gave a 40% cure rate as measured by survival of the animals for 60 days. Other tests with $cis$-Pt(NH$_3$)$_2$ complexed with (PGA) using four animals per dose gave a 75% cure rate at 20 mg Pt/kg dose and 50% cure rate at 15 mg Pt/kg dose. Mice (four animals per dose level) treated with either 10 mg Pt/kg or 20 mg Pt/kg of $cis$-Pt(NH$_3$)$_2$ complexed with (PGA) on days 1, 3 and 6 showed cure rates of 100%. After 60 days, blood from surviving animals was administered to other uninfected animals, and no infection resulted. In the testing regime, cisplatin was ineffective at all doses.

Example VIII

Various water- and plasma-soluble compounds of the invention were screened for anticancer activity in mice using *ascites* P388 leukemia cells. The mice were CDFl, female, weighing approximately 20 grams. In each test animal, about $4.8 \times 10^6$ cells were implanted intraperitoneally. The test drugs were administered intraperitoneally one day after infection. The results are shown in the following Table I.

TABLE I

| Treatment | No. animals | Dosage mg Pt/kg | Median | | Log$_{10}$ change in tumour burden at end Rx |
|---|---|---|---|---|---|
| | | | Day of death | % ILS | |
| Negative control (no treatment) | 20 | — | 10 | | |
| cis-Pt(DACH)(PGA) complex | 3 | 96 | 16 | +60 | −4.1 |
| | 3 | 48 | 17 | +70 | −4.8 |
| | 3 | 24 | 17 | +70 | −4.8 |
| | 3 | 12 | 14 | +40 | −2.7 |
| cis-Pt(DACH) (Bovine serum albumin) complex | 3 | 96 | 14 | +40 | −2.7 |
| | 3 | 48 | 11 | +10 | −0.7 |
| | 3 | 24 | 11 | +10 | −0.7 |
| | 3 | 12 | 11 | +10 | −0.7 |
| cis-Pt(cyclohexylamine)$_2$ (PGA) complex | 3 | 96 | 13 | +30 | −2.1 |
| | 3 | 48 | 12 | +20 | −1.4 |
| | 3 | 24 | 11 | +10 | −0.7 |
| | 3 | 12 | 11 | +10 | −0.7 |
| cis-Pd(en) (PGA) complex | 3 | 96 | Toxic | | — |
| | 3 | 48 | Toxic | | — |
| | 3 | 24 | 14 | +40 | −2.7 |
| | 3 | 12 | 10 | 0 | — |
| cis-Pd(NH$_3$)$_2$ (PGA) complex | 3 | 96 | Toxic | | |
| | 3 | 48 | 14 | +40 | −2.7 |
| | 3 | 24 | 10 | 0 | 0 |
| | 3 | 12 | 9 | −10 | +0.7 |
| cis-Pd(DACH) (PGA) complex | 3 | 96 | Toxic | | |
| | 3 | 48 | Toxic | | |
| | 3 | 24 | 11 | +10 | −0.7 |
| | 3 | 12 | 10 | 0 | 0 |
| cis-Pt(DACH) (dextran sulphate) complex | 3 | 6 | 22 | +100 | −6.7 |
| | 3 | 3 | 20 | +81 | −6.2 |
| | 3 | 1.5 | 18 | +63 | −4.8 |
| Negative control (no treatment) | { 20 | — | 11 | | |
| cis-Pt(NH$_3$)$_2$ (PGA) complex | 10 | 56 | Toxic | | |
| | 10 | 38 | 17 | +54 | −4.5 |
| | 10 | 25 | 16.5 | +50 | −4.2 |
| | 10 | 17 | 14 | +27 | −2.3 |
| | 10 | 11 | 13 | +18 | −1.5 |
| Positive control (cisplatin) | 10 | 5.2 | Toxic | | |
| | 10 | 3.5 | 18.5 | +68 | −5.7 |
| | 10 | 2.3 | 16.5 | +50 | −4.2 |
| Negative control (no treatment) | { 20 | — | 11 | | |

Note:
　　Each test series is separated by a line.
　　%ILS=Increase in life span.

In Table I the column headed "Log$_{10}$ Change in Tumour Burden at End Rx" means the net log change in viable tumour cell population at the end of the procedure as compared to the start and forms a convenient measure of the order of magnitude of the cell population's change due to treatment. Thus a "−6 log change" means a 99.9999% reduction.

The results achieved as described are considered to demonstrate that compounds produced in

5

accordance with the invention are in many cases more tolerable in mammalian test bodies than the standard cisplatin. It was observed that the composition of *cis*-Pt (cyclohexylamine)$_2$ with ferritin, made in manner similar to the procedure described in Example VI, was extremely toxic in mice when administered as described above.

The therapeutic value of certain of the water soluble drugs in trypanosomal treatment is high. It appears that the enhanced solubility characteristics of the compounds will be of great benefit. The degree of solubility and biological parameters can be controlled by varying the total concentration of aminated platinum or palladium complexed to the anionic macromolecules.

The following examples relate to the water-insoluble compositions of matter of the present invention.

Example IX

One gram of the sodium salt of poly-L-glutamic acid was dissolved in 20 ml of distilled water in a round bottom flask fitted with a magnetic stirrer. To this flask was then slowly added 10.5 ml of an aqueous solution of *cis*-Pt(NH$_3$)$_2$(NO$_3$)$_2$ . 2H$_2$O containing 0.495 g/ml of platinum while stirring. A product slowly precipitated from the stirred solution but redissolved upon the addition of 10 ml of distilled water. An additional 2.1 ml of the platinum solution was added to provide a precipitated product of crosslinked nature. After filtration, washing and drying the thus produced composition of matter contained 33.68% platinum which calculates out to 86.0% of the carboxylic acid groups on the side chains of the poly-L-glutamic acid being complexed with the cis-[Pt(NH$_3$)$_2$]$^{++}$ moiety.

Example X

In a preparation similar to that described in Example IX, 0.5 g of the sodium salt of poly-L-glutamic acid was reacted with 0.312 g of platinum in the form of the *cis*-Pt(NH$_3$)$_2$(NO$_3$)$_2$ . 2H$_2$O compound to provide a product containing 38.55% platinum. This equates to a reaction of 95.1% of the carboxylic acid units on the poly-L-glutamic acid with the cis-[Pt(NH$_3$)$_2$]$^{++}$ moiety.

Example XI

0.857 g of sodium carboxymethyl cellulose was dissolved in a mixture of 60 ml of water and 20 ml of dimethyl formamide. The containing flask was covered with aluminium foil and 9.6 ml of an aqueous solution of *cis*-Pt(NH$_3$)$_2$(NO$_3$)$_2$ . 2H$_2$O was added while stirring. After an hour product began to precipitate. Subsequently product was collected, vacuum filtered, washed with water and acetone and dried under vacuum. The thus produced product analyzed 24.8% platinum which equates to reaction of 88.2% of the free carboxylic acid groups of the carboxymethyl cellulose complexing with the *cis*-[Pt(NH$_3$)$_2$]$^{++}$ moiety.

Example XII

The composition of matter as prepared in Example IX was tested for activity against *ascites* P388 leukemia in CDFI female mice. At a non-toxic dose level of 30 mg/kg of mouse weight, administered intraperitoneally as a single dose on the day following implantation of 10$^6$ of tumour cells, the composition of Example IX provided a 36% increase in life span and an approximate −3.0 log$_{10}$ change in tumour burden at the end of the test. Toxicity data on the composition of matter of Example IX derived from non-tumour bearing CDFI female mice indicated LD$_{10}$, LD$_{50}$ and LD$_{90}$ of 68, 99 and 141 mg of platinum/kg of mouse respectively.

**Claims for the Contracting States: BE, CH, DE, FR, GB, IT, LI, LU, NL, SE**

1. Platinum and palladium compositions comprising water-soluble anionic organic macromolecules, namely anionic polyamino acids, anionic polysaccharides or enzymes having anionic functionalities, having a molecular weight from 5000 to 60 000, combined with a moiety of square planar coordination about the metal atom M, having the formula [*cis* MLL']$^{++}$, wherein M is divalent platinum or divalent palladium and L and L' are ammonia or mono- or di-functional primary amines, so as to satisfy at least part of the valence requirements of the anionic functional groups of the anionic macromolecules.

2. Compositions according to claim 1 in which the valence requirements of at least 80% of the anionic functional groups of the anionic macromolecules are satisfied and the compositions are insoluble in water.

3. Compositions according to claim 1 or claim 2 in which the macromolecules are poly-L-glutamic acid, poly-L-aspartic acid, serum albumin, chondroitin sulphate, dextran sulphate, carboxymethylcellulose, poly[divinylether-co-maleic anhydride], poly[acrylic acid-co-maleic anhydride], polyacrylic acid, ferritin or desulphated heparin.

4. Compositions according to any preceding claim in which the groups L and L' are ammonia, 1,2-diamino-cyclohexane or ethylenediamine.

5. Compositions according to claim 1 in which M is platinum, L and L' are ammonia and the macromolecule is poly-L-glutamic acid.

6. Compositions according to claim 1 in which M is platinum, L and L' are the amine functions of 1,2-diaminocyclohexane and the macromolecule is poly-L-glutamic acid or dextran sulphate.

7. Compositions according to claim 6 that have been lyophilized.

8. Compositions according to claim 2 in which M is platinum, L and L' are ammonia and the macromolecule is poly-L-glutamic acid or carboxymethylcellulose.

9. Method of making a composition according to claim 1 which comprises reacting, in aqueous solution, the anionic macromolecules with the compounds $cis$-MLL'$(H_2O)_2(NO_3)_2$.

10. Method of making a composition according to claim 4 which comprises reacting, in aqueous solution, the anionic macromolecules with the compound $cis$-MLL'$(H_2O)_2(NO_3)_2$ wherein L and L' are ammonia, 1,2-diaminocyclohexane or ethylenediamine.

11. A composition according to any one of claims 1 to 8 for use as a medicament for the treatment of cancer, tumors or trypanosomiasis.

12. A water-insoluble composition according to any one of claims 1 to 8 for use in the treatment of tumors.

13. A water-soluble composition according to any one of claims 1 to 8 for use in the treatment of trypanosomiasis.

## Claims for the Contracting State: AT

1. Method of making a platinum or palladium composition which comprises reacting in aqueous solution water-soluble anionic organic macromolecules, namely anionic polyamino acids, anionic polysaccharides or enzymes having anionic functionalities, having a molecular weight of from 5000 to 60 000, with an aminated $cis$-platinum or $cis$-palladium compound in aquated nitrate form MLL'$(H_2O)_2(NO_3)_2$, wherein M is divalent Pt or Pd and L and L' are ammonia or mono- or di-functional primary amines, so as to satisfy at least part of the valence requirements of the anionic functional groups of the anionic macromolecules.

2. Method according to claim 1 in which the valence requirements of at least 80% of the anionic functional groups of the anionic macromolecules are satisfied and the composition is insoluble in water.

3. Method according to claim 1 or claim 2 in which the macromolecules are poly-L-glutamic acid, poly-L-aspartic acid, serum albumin, chondroitin sulphate, dextran sulphate, carboxymethylcellulose, poly[divinylether-comaleic anhydride], poly[acrylic acid-co-maleic anhydride], polyacrylic acid, ferritin or desulphated heparin.

4. Method according to any preceding claim in which the groups L and L' are ammonia, 1,2-diaminocyclohexane or ethylenediamine.

5. Method according to claim 1 in which M is platinum, L and L' are ammonia and the macromolecule is poly-L-glutamic acid.

6. Method according to claim 1 in which M is platinum, L and L' are the amine functions of 1,2-diaminocyclohexane and the macromolecule is poly-L-glutamic acid or dextran sulphate.

7. Method according to claim 6 which includes the step of lyophilizing the composition.

8. Method according to claim 1 in which M is platinum, L and L' are ammonia and the macromolecule is poly-L-glutamic acid or carboxymethyl cellulose.

9. Method of making a platinum or palladium composition for use as a medicament for the treatment of cancers, tumours or trypanosomiasis, which comprises reacting in aqueous solution water-soluble anionic organic macromolecules, namely anionic polyamino acids, anionic polysaccharides or enzymes having anionic functionalities, having a molecular weight of from 5000 to 60 000, with an aminated $cis$-platinum or $cis$-palladium compound in aquated nitrate form MLL'$(H_2O)_2(NO_3)_2$, wherein M is divalent Pt or Pd and L and L' are ammonia or mono- or difunctional primary amines, so as to satisfy at least part of the valence requirements of the anionic functional groups of the anionic macromolecules.

10. Method of making a water-insoluble platinum or palladium composition for use in the treatment of tumours, which comprises reacting in aqueous solution water-soluble anionic organic macromolecules, namely anionic polyamino acids, anionic polysaccharides, or enzymes having anionic functionalities, having a molecular weight of from 5000 to 60 000, with an aminated $cis$-platinum or $cis$-palladium compound in aquated nitrate form MLL'$(H_2O)_2(NO_3)_2$, wherein M is divalent Pt or Pd and L and L' are ammonia or mono- or di-functional primary amines, so as to satisfy the valence requirements of at least 80% of the anionic functional groups of the anionic macromolecules.

11. Method of making a water-soluble composition for use in the treatment of trypanosomiasis, which comprises reacting in aqueous solution water-soluble anionic organic macromolecules, namely anionic polyamino acids, anionic polysaccharides or enzymes having anionic functionalities, having a molecular weight of from 5000 to 60 000, with an aminated $cis$-platinum or $cis$-palladium compound in aquated nitrate form MLL'$(H_2O)_2(NO_3)_2$, wherein M is divalent Pt or Pd and L and L' are ammonia or mono- or di-functional primary amines, so as to satisfy the valence requirements of less than 80% of the anionic functional groups of the anionic macromolecules.

## Patentansprüche für die Vertragsstaaten: BE, CH, DE, FR, GB, IT, LI, LU, NL, SE

1. Platin- und Palladiumzusammensetzungen umfassend wasserlösliche anionische organische Makromoleküle, nämlich anionische Polyaminosäuren, anionische Polysaccharide oder Enzyme mit anionischer Funktionalität, mit einem Molekulargewicht von 5000 bis 60,000, kombiniert mit einem Teil

# 0 111 388

einer quadratischen planaren Anordnung bezüglich des Metallatoms M mit der Formel [*cis* MLL']$^{++}$, in der M bivalentes Platin oder bivalentes Palladium ist und L und L' Ammoniak oder mono- oder bifunktionelle primäre Amine sind, sodaß zumindest ein Teil der Valenzerfordernisse der anionischen funktionellen Gruppen der anionischen Makromoleküle abgesättigt ist.

2. Zusammensetzungen nach Anspruch 1, in denen die Valenzerfordernisse von mindestens 80% der anionischen funktionellen Gruppen der anionischen Makromoleküle abgesättigt sind und die Zusammensetzungen in Wasser unlöslich sind.

3. Zusammensetzungen nach Anspruch 1 oder 2, in denen die Makromoleküle Poly-L-Glutaminsäure, Poly-L-Asparaginsäure, Serumalbumin, Chondroitinsulfat, Dextransulfat, Carboxymethylcellulose, Poly[divinylether-co-maleinanhydrid], Poly[acrylsäure-co-maleinanhydrid], Polyacrylsäure, Ferritin und desulfatiertes Heparin.

4. Zusammensetzungen nach einem der vorhergehenden Ansprüche, in denen die Gruppen L und L' Ammoniak, 1,2-Diaminocyclohexan oder Ethylendiamin sind.

5. Zusammensetzungen nach Anspruch 1, in denen M Platin, L und L' Ammoniak und das Makromolekül Poly-L-Glutaminsäure sind.

6. Zusammensetzung nach Anspruch 1, in denen M Platin, L und L' die Aminfunktionen von 1,2-Diaminocyclohexan und das Makromolekül Poly-L-Glutaminsäure oder Dextransulfat sind.

7. Zusammensetzungen nach Anspruch 6, die lyophilisiert wurden.

8. Zusammensetzungen nach Anspruch 2, in denen M Platin, L und L' Ammoniak und das Makromolekül Poly-L-Glutaminsäure oder Carboxymethylcellulose sind.

9. Verfahren zur Herstellung einer Zusammensetzung nach Anspruch 1, bei dem in wässriger Lösung, die anionischen Makromoleküle mit der Verbindung *cis*-MLL'(H$_2$O)$_2$(NO$_3$)$_2$ zur Reaktion gebracht werden.

10. Verfahren zur Herstellung einer Zusammensetzung nach Anspruch 4, bei dem, in wässriger Lösung, die anionischen Makromoleküle mit der Verbindung *cis*-MLL'(H$_2$O)$_2$(NO$_3$)$_2$ zur Reaktion gebracht werden, in der L und L' Ammoniak, 1,2-Diaminocyclohexan oder Ethylendiamin sind.

11. Eine Zusammensetzung nach einem der Ansprüche 1 bis 8 für die Verwendung als Medikament zur Behandlung von Krebs, Tumoren oder Trypanosomiasis.

12. Eine wasserunlösliche Zusammensetzung nach einem der Ansprüche 1 bis 8, zur Verwendung in der Behandlung von Tumoren.

13. Eine wasserlösliche Zusammensetzung nach einem der Ansprüche 1 bis 8 zur Verwendung in der Behandlung von Trypanosomiasis.

## Patentansprüche für den Vertragsstaat: AT

1. Verfahren zur Herstellung einer Platin- oder Palladiumzusammensetzung, bei dem, in wässriger Lösung, wasserlösliche anionische organische Makromoleküle, nämlich anionische Polyaminosäuren, anionische Polysaccharide oder Enzyme mit anionischer Funktionalität, mit einem Molekulargewicht vo 5000 bis 60 000, mit einer aminierten *cis*-Platin- oder *cis*-Palladiumverbindung in wässriger Nitratform MLL'(H$_2$O)$_2$(NO$_3$)$_2$ zur Reaktion gebracht werden, in der M bivalentes Pt oder Pd und L und L' Ammoniak oder mono- oder bifunktionelle primäre Amine sind, sodaß zumindest ein Teil der Valenzerfordernisse der anionischen funktionellen Gruppen der anionischen Makromoleküle abgesättigt ist.

2. Verfahren nach Anspruch 1, in denen die Valenzerfordernisse von mindestens 80% der anionischen funktionellen Gruppen der anionischen Makromoleküle abgesättigt sind und die Zusammensetzung in Wasser unlöslich ist.

3. Verfahren nach Anspruch 1 oder 2, in denen die Makromoleküle Poly-L-Glutaminsäure, Poly-L-Asparaginsäure, Serumalbumin, Chondroitinsulfat, Dextransulfat, Carboxymethylcellulose, Poly[divinylether-co-maleinanhydrid], Poly[acrylsäure-co-maleinanhydrid], Polyacrylsäure, Ferritin und desulfatiertes Heparin.

4. Verfahren nach einem der vorhergehenden Ansprüche, in denen die Gruppen L und L' Ammoniak, 1,2-Diaminocyclohexan oder Ethylendiamin sind.

5. Verfahren nach Anspruch 1, in denen M Platin, L und L' Ammoniak und das Makromolekül Poly-L-Glutaminsäure sind.

6. Verfahren nach Anspruch 1, in denen M Platin, L und L' die Aminfunktionen von 1,2-Diaminocyclohexan und das Makromolekül Poly-L-Glutaminsäure oder Dextransulfat sind.

7. Verfahren nach Anspruch 6, das den Schritt zum Lyophilisieren der Verbindung umfaßt.

8. Verfahren nach Anspruch 1, bei dem M Platin, L und L' Ammoniak und das Makromolekül Poly-L-Glutaminsäure oder Carboxymethylcellulose sind.

9. Verfahren zur Herstellung einer Platin- oder Palladiumzusammensetzung für die Verwendung als Medikament zur Behandlung von Krebs, Tumoren oder Tyrpanosomiasis, bei dem, in wässriger Lösung, wasserlösliche anionische organische Makromoleküle, nämlich anionische Polyaminosäuren, anionische Polysaccharide oder Enzyme mit anionischer Funktionalität, mit einem Molekulargewicht von 5000 bis 60 000, mit einer aminierten *cis*-Platin- oder *cis*-Palladiumverbindung in wässriger Nitratform MLL'(H$_2$O)$_2$(NO$_3$)$_2$ zur Reaktion gebracht werden, in der M bivalentes Pt oder Pd und L und L' Ammoniak oder mono- oder bifunktionelle primäre Amine sind, sodaß zumindest ein Teil der Valenzerfordernisse der anionischen funktionellen Gruppen der anionischen Makromoleküle abgesättigt ist.

10. Verfahren zur Herstellung einer Platin- oder Palladiumzusammensetzung zur Verwendung in der Behandlung von Tumoren, bei dem, in wässriger Lösung, wasserlösliche anionische organische Makromoleküle, nämlich anionische Polyaminosäuren, anionische Polysaccharide oder Enzyme mit anionischer Funktionalität, mit einem Molekulargewicht von 5000 bis 60 000, mit einer aminierten cis-Platin- oder cis-Palladiumverbindung in wässriger Nitratform MLL'(H$_2$O)$_2$(NO$_3$)$_2$ zur Reaktion gebracht werden, in der M bivalentes Pt oder Pd und L und L' Ammoniak oder mono- oder bifunktionelle primäre Amine sind, sodaß die Valenzerfordernisse von mindestens 80% der anionischen funktionellen Gruppen der anionischen Makromoleküle abgesättigt sind.

11. Verfahren zur Herstellung einer Platin- oder Palladiumzusammensetzung zur Verwendung in der Behandlung von Trypanosomiasis, bei dem, in wässriger Lösung, wasserlösliche anionische organische Makromoleküle, nämlich anionische Polyaminosäuren, anionische Polysaccharide oder Enzyme mit anionischer Funktionalität, mit einem Molekulargewicht von 5000 bis 60 000, mit einer aminierten cis-Platin- oder cis-Palladiumverbindung in wässriger Nitratform MLL'(H$_2$O)$_2$(NO$_3$)$_2$ zur Reaktion gebracht werden, in der M bivalentes Pt oder Pd und L und L' Ammoniak oder mono- oder bifunktionelle primäre Amine sind, sodaß die Valenzerfordernisse von weniger als 80% der anionischen funktionellen Gruppen der anionischen Makromoleküle erfüllt sind.

**Revendications pour les Etats Contractants: BE, CH, DE, FR, GB, IT, LI, LU, NL, SE**

1. Compositions de platine et de palladium comprenant des macromolécules organiques anioniques solubles dans l'eau, à savoir des polyamino-acides anioniques, des polysaccharides anioniques ou des enzymes ayant des fonctionnalités anioniques ayant un poids moléculaire allant de 5000 à 60000 combinées avec une fraction de coordination plane carrée autour de l'atome de métal M, ayant la formule [cis MLL']$^{++}$, où M est le platine bivalent ou le palladium bivalent et L et L' représentent l'ammoniac ou des amines primaires mono- ou bifonctionnelles, de manière à satisfaire au moins une partie des exigences en matière de valence des groupes fonctionnels anioniques des macromolécules anioniques.

2. Compositions selon la revendication 1 où les exigences en matière de valence d'au moins 80% des groupes fonctionnels anioniques des macromolécules anioniques sont satisfaites et les compositions sont insolubles dans l'eau.

3. Compositions selon les revendications 1 et 2 où les macromolécules sont l'acide poly-L-glutamique, l'acide poly-L-aspartique, la sérum-albumine, le sulfate de chondroïtine, le sulfate de dextran, la carboxyméthylcellulose, le poly[divinyléther-co-anhydride maléique], le poly[acide acrylique-co-anhydride maléique], l'acide polyacrylique, la ferritine ou l'héparine désulfatée.

4. Compositions selon l'une quelconque des revendications précédentes où les groupes L et L' représentent l'ammoniac, le 1,2-diamino-cyclohexane ou l'éthylènediamine.

5. Compositions selon la revendication 1 où M est le platine, L et L' représentent l'ammoniac et la macromolécule est l'acide poly-L-glutamique.

6. Compositions selon la revendication 1 où M est le platine, L et L' sont les fonctions amine du 1,2-diaminocyclohexane et la macromolécule est l'acide poly-L-glutamique ou le sulfate de dextran.

7. Compositions selon la revendication 6 qui ont été lyophilisées.

8. Compositions selon la revendication 2 où M est le platine, L et L' représentent l'ammoniac et la macromolécule est l'acide poly-L-glutamique ou la carboxyméthylcellulose.

9. Procédé de préparation d'une composition selon la revendication 1 dans lequel on fait réagir, en solution aqueuse, les macromolécules anioniques avec le composé cis-MLL'(H$_2$O)$_2$(NO$_3$)$_2$.

10. Procédé de préparation d'une composition selon la revendication 4 dans lequel on fait réagir, en solution aqueuse, les macromolécules anioniques avec le composé cis-MLL'(H$_2$O)$_2$(NO$_3$)$_2$ où L et L' représentent l'ammoniac, le 1,2-diaminocyclohexane ou l'éthylènediamine.

11. Composition selon l'une quelconque des revendications 1 à 8 pour application comme médicament pour le traitement du cancer, des tumeurs ou de la trypanosomiase.

12. Composition insoluble dans l'eau selon l'une quelconque des revendications 1 à 8 pour application dans le traitement des tumeurs.

13. Composition soluble dans l'eau selon l'une quelconque des revendications 1 à 8 pour application dans le traitement de la trypanosomiase.

**Revendications pour l'Etat Contractant: AT**

1. Procédé de préparation d'une composition de platine ou de palladium dans lequel on fait réagir en solution aqueuse des macromolécules organiques solubles dans l'eau, à savoir des polyamino-acides anioniques, des polysaccharides anioniques ou des enzymes ayant des fonctionnalités anioniques, ayant un poids moléculaire allant de 5 000 à 60 000, avec un composé de cis-platine ou de cis-palladium aminé sous forme de nitrate avec molécules d'eau MLL'(H$_2$O)$_2$(NO$_3$)$_2$, où M est le Pt ou Pd bivalent et L et L' représentent l'ammoniac ou des amines primaires mono- ou difonctionnelles, de manière à satisfaire au moins une partie des exigences en matière de valence des groupes fonctionnels anioniques des macromolécules anioniques.

2. Procédé selon la revendication 1 où les exigences en matière de valence d'au moins 80% des groupes fonctionnels anioniques des macromolécules anioniques sont satisfaites et la composition est insoluble dans l'eau.

3. Procédé selon les revendications 1 et 2 où les macromolécules sont l'acide poly-L-glutamique, l'acide poly-L-aspartique, la sérum-albumine, le sulfate de chondroïtine, le sulfate de dextran, la carboxyméthylcellulose, le poly[divinyléther-co-anhydride maléique], le poly[acide acrylique-co-anhydride maléique], l'acide polyacrylique, la ferritine ou l'héparine désulfatée.

4. Procédé selon l'une quelconque des revendications précédentes où les groupes L et L' représentent l'ammoniac, le 1,2-diaminocyclohexane ou l'éthylènediamine.

5. Procédé selon la revendication 1 où M est le platine, L et L' représentent l'ammoniac et la macromolécule est l'acide poly-L-glutamique.

6. Procédé selon la revendication 1 où M est le platine, L et L' représentent les fonctions amine du 1,2-diaminocyclohexane et la macromolécule est l'acide poly-L-glutamique ou le sulfate de dextran.

7. Procédé selon la revendication 6 qui comprend l'étape de lyophilisation de la composition.

8. Procédé selon la revendication 1 où M est le platine, L et L' représentent l'ammoniac et la macromolécule est l'acide poly-L-glutamique ou la carboxyméthyl-cellulose.

9. Procédé de préparation d'une composition de platine ou de palladium pour application comme médicament pour le traitement des cancers, des tumeurs ou de la trypanosomiase, dans lequel on fait réagir en solution aqueuse des macromolécules organiques anioniques solubles dans l'eau, à savoir des polyamino-acides anioniques, des polysaccharides anioniques ou des enzymes ayant des fonctionnalités anioniques, ayant un poids moléculaire allant de 5 000 à 60 000, avec un composé de *cis*-platine ou de *cis*-palladium aminé sous forme de nitrate avec molécules d'eau $MLL'(H_2O)_2(NO_3)_2$, où M et le Pt ou Pd bivalent et L et L' représentent l'ammoniac ou des amines primaires mono- ou bifonctionnelles, de manière à satisfaire au moins une partie des exigences en matière de valence des groupes fonctionnels anioniques des macromolécules anioniques.

10. Procédé de préparation d'une composition de platine ou de palladium insoluble dans l'eau pour application dans le traitement des tumeurs, dans lequel on fait réagir en solution aqueuse des macromolécules organiques anioniques solubles dans l'eau, à savoir des polyamino-acides anioniques, des polysaccharides anioniques ou des enzymes ayant des fonctionnalités anioniques, ayant un poids moléculaire allant de 5 000 à 60 000, avec un composé de *cis*-platine ou de *cis*-palladium aminé sous forme de nitrate avec molécules d'eau $MLL'(H_2O)_2(NO_3)_2$, où M est le Pt ou Pd bivalent et L et L' représentent l'ammoniac ou des amines primaires mono- ou bifonctionnelles, de manière à satisfaire aux exigences en matière de valence d'au moins 80% des groupes fonctionnels anioniques des macromolécules anioniques.

11. Procédé de préparation d'une composition soluble dans l'eau pour application dans le traitement de la trypanosomiase, dans lequel on fait réagir en solution aqueuse des macromolécules organiques anioniques solubles dans l'eau, à savoir des polyamino-acides anioniques, des polysaccharides anioniques ou des enzymes ayant des fonctionnalités anioniques, ayant un poids moléculaire allant de 5 000 à 60 000, avec un composé de *cis*-platine ou de *cis*-palladium sous forme de nitrate avec molécules d'eau $MLL'(H_2O)_2(NO_3)_2$, où M est le Pt ou le Pd bivalent et L et L' représentent l'ammoniac ou des amines primaires mono- ou bifonctionnelles, de manière à satisfaire aux exigences en matière de valence de moins de 80% des groupes fonctionnels anioniques des macromolécules anioniques.